# EUROPEAN PATENT APPLICATION

(11) **EP 4 840 844 A1**
(43) Date of publication of application: **18.11.2026**
(21) Application number: 25774799.8
(22) Date of filing: 19.03.2025
(51) Int. Cl.: C07C 263/18, C07C 265/14, C07C 265/08, C07C 263/10, C07C 263/20, C08G 18/76, G02B 1/04

(54) **ISOCYANATE COMPOSITION AND POLYMERIZABLE COMPOSITION COMPRISING SAME**

(30) Priority: 19.03.2024 KR 20240038067
(71) Applicant: Hanwha Solutions Corporation, Jung-gu Seoul 04541 (KR)
(72) Inventor: SIM, Yujin, Daejeon 34128 (KR); WOO, Eun Ji, Daejeon 34128 (KR); KIM, Jiyeon, Daejeon 34128 (KR); KIM, Jong Jin, Daejeon 34128 (KR); JEON, Ju Eun, Daejeon 34128 (KR); KWON, Dowoo, Daejeon 34128 (KR)
(74) Representative: Berggren Oy
(86) International application number: PCT/KR2025/003562
(87) International publication number: WO 2025/198327

(57) **Abstract**

Provided are an isocyanate composition with improved storage stability even for a long period of several months or more, suppressing the occurrence of discoloration and cloudiness, and capable of improving transparency when applied to optical products, and a polymerizable composition comprising the same.

## Description

### [TECHNICAL FIELD]

### CROSS-REFERENCE TO RELATED APPLICATION(S)

This application claims the benefit of priority to Korean Patent Application No. 10-2024-0038067, filed on March 19, 2024, and Korean Patent Application No. 10-2025-0035252, filed on March 19, 2025, the disclosures of which are incorporated herein by reference in their entirety.

The present disclosure relates to an isocyanate composition and a polymerizable composition comprising the same, which exhibit improved storage stability even for a long period of several months or more, inhibit the occurrence of discoloration and cloudiness, maintain excellent discoloration resistance when applied to optical products, and improve transparency.

### [BACKGROUND]

Isocyanate-based compounds are compounds with high utility value not only in the chemical and resin industries but also as fine chemical products, including optical materials. Xylylene diisocyanate (hereinafter referred to as XDI), a representative example of isocyanate-based compounds, is increasingly in demand as a high-value-added chemical material serving as a raw material for poly(thio)urethane used in high-end optical lenses and flexible displays.

However, because isocyanate-based compounds exhibit high reactivity, they are prone to discoloration or cloudiness due to reaction with oxygen or moisture in the atmosphere during storage. Furthermore, when an isocyanate-based compound is stored for a long period of several months or more, it forms oligomers such as dimers (e.g., uretdione) and trimers through self-polymerization, which leads to issues such as differences in polymerizability and problems such as discoloration and cloudiness in product applications.

Isocyanate-based compounds are widely used as raw materials for polyurethanes in coatings, pressure-sensitive/adhesives, paints, foams, and optical materials. When a polyurethane lens is manufactured using an isocyanate-based compound in which such discoloration or cloudiness has occurred, the rapid increase in the molecular weight of the polymerization liquid causes a decrease in stirring efficiency, an increase in filter time, and filter clogging, resulting in reduced workability. Additionally, non-uniform polymerization of the manufactured lens leads to issues such as striae, reduced transparency, and discoloration.

To solve these problems, although XDI is packaged in a nitrogen-substituted state during the product packaging step to ensure storage stability, the product is often not fully consumed at once, leading to the possibility of exposure to the atmosphere, which frequently causes discoloration or cloudiness of XDI.

Accordingly, research and development are continuously underway for an isocyanate composition that is free from the risk of discoloration or cloudiness even during long-term storage due to improved storage stability, and that can maintain excellent discoloration resistance and improve transparency when manufacturing optical articles such as lenses.

### [DETAILED DESCRIPTION OF THE INVENTION]

### [Technical Problem]

The present disclosure aims to provide an isocyanate composition that is free from the risk of discoloration or cloudiness even during long-term storage of several months or more due to improved storage stability, and that can improve the transparency of a lens when used as a raw material for optical articles such as poly(thio)urethane lenses.

Furthermore, the present disclosure aims to provide a polymerizable composition comprising the above-described isocyanate composition and an optical article comprising a polymer obtained therefrom.

### [Technical Solution]

According to one embodiment of the present disclosure, there is provided an isocyanate composition comprising xylylene diisocyanate and a compound represented by the following Chemical Formula 1, wherein the content of said compound is greater than 60 ppm to 200 ppm or less:

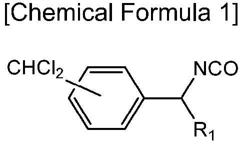

In Chemical Formula 1,
R₁ is hydrogen, methyl, or ethyl.

In one embodiment, the content of the compound represented by Chemical Formula 1 in the isocyanate composition may be greater than 62 ppm to 180 ppm or less.

In one embodiment, the isocyanate composition may have a chlorine (CI) content of 200 ppm or less based on the total weight of the isocyanate composition, measured using a combustion ion chromatograph in accordance with the American Society for Testing and Materials (ASTM) D 7359 method.

In one embodiment, the isocyanate composition may have an APHA color index of 0.1 or more to 25 or less, measured using a spectrophotometer (Ultrascan Pro by HunterLab, light source: C/2) in accordance with the American Society for Testing and Materials (ASTM) E313 method.

In one embodiment, the isocyanate composition may further comprise at least one selected from the group consisting of chloromethylbenzyl isocyanate, isocyanomethylbenzaldehyde, and isocyanomethylbenzonitrile.

In one embodiment, the content of uretdione in the isocyanate composition, measured after 12 months of storage, may be 5 parts by weight or less based on 100 parts by weight of the isocyanate composition.

According to another embodiment of the present disclosure, there is provided a method for producing an isocyanate composition, comprising: reacting xylylene diamine or a xylylene diamine salt with phosgene to obtain a reaction mixture; and purifying the reaction mixture under reduced pressure at a temperature of greater than 120 °C and less than 230 °C.

According to another embodiment of the present disclosure, there is provided a polymerizable composition comprising the above-described isocyanate composition; and at least one selected from the group consisting of a polyfunctional thiol-based compound, a polyfunctional alcohol-based compound, and a polyfunctional episulfide-based compound.

The polymerizable composition may further comprise at least one selected from the group consisting of a polymerization initiator, an internal release agent, and an ultraviolet absorber.

Furthermore, according to another embodiment of the present disclosure, there is provided a composition for polyisocyanate polymerization, comprising the above-described isocyanate composition; and at least one polyfunctional monomer compound selected from the group consisting of a polyfunctional thiol-based compound, a polyfunctional alcohol-based compound, and a polyfunctional episulfide-based compound.

Furthermore, according to another embodiment of the present disclosure, there is provided a polyisocyanate composition comprising a polyisocyanate obtained by polymerizing the above-described isocyanate composition and a polyfunctional alcohol-based compound.

Furthermore, according to another embodiment of the present disclosure, there is provided a polyisocyanate composition comprising a polyisocyanate obtained by polymerizing the above-described isocyanate composition and a polyfunctional thiol-based compound or a polyfunctional episulfide-based compound.

According to another embodiment of the present disclosure, there is provided an optical article comprising a polymer obtained by polymerizing the above-described polymerizable composition.

### [ADVANTAGEOUS EFFECTS]

The isocyanate composition according to the present disclosure has improved storage stability, thereby inhibiting the occurrence of discoloration or cloudiness even during long-term storage of several months or more, and can improve transparency when applied to optical products. Furthermore, a polymer prepared by polymerizing the isocyanate composition with a polyfunctional thiol-based compound, a polyfunctional alcohol-based compound, or a polyfunctional episulfide-based compound also exhibits excellent transparency, making it useful for manufacturing articles such as optical pressure-sensitive adhesives, optical adhesives, optical coatings, and optical lenses.

### [DETAILED DESCRIPTION OF THE EMBODIMENTS]

The terminology used herein is for the purpose of describing specific embodiments only and is not intended to be limiting of the present invention. The singular forms include the plural forms as well, unless the context clearly indicates otherwise. In this disclosure, the terminology "include," "comprise," or "have" is used to describe stated features, steps, components, or combinations thereof, and does not preclude the addition of one or more other features, numbers, steps, components, or combinations thereof.

As the present invention can be variously modified and have various forms, specific embodiments thereof are shown by way of examples and will be described in detail. However, it is not intended to limit the present invention to the particular form disclosed and it should be understood that the present invention includes all modifications, equivalents, and replacements within the idea and technical scope of the present invention.

Hereinafter, the present disclosure will be described in detail.

### Isocyanate Composition

The isocyanate composition of the present disclosure comprises xylylene diisocyanate and a compound represented by the following Chemical Formula 1, satisfying a content of the compound represented by Chemical Formula 1 of greater than 60 ppm to 200 ppm or less:

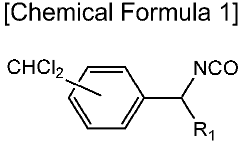

In Chemical Formula 1,
R₁ is hydrogen, methyl, or ethyl.

Xylylene diisocyanate (XDI) is the main component of the isocyanate composition according to an embodiment of the present disclosure. Specifically, the xylylene diisocyanate may be at least one selected from the group consisting of 1,2-xylylene diisocyanate (o-xylylene diisocyanate, o-XDI), 1,3-xylylene diisocyanate (m-xylylene diisocyanate, m-XDI), and 1,4-xylylene diisocyanate (p-xylylene diisocyanate, p-XDI). Preferably, the xylylene diisocyanate may be m-XDI.

As the main component of the isocyanate composition, the content of xylylene diisocyanate in the isocyanate composition may be 97.7 area% or more, 98.0 area% or more, 98.3 area% or more, 99.0 area% or more, or 99.2 area% or more, and 99.9 area% or less, based on GC area % analysis.

Meanwhile, the xylylene diisocyanate (XDI) included in the isocyanate composition may be a commercially available product or may be prepared using one of various synthesis methods known in the art; the specific synthesis method is not particularly limited and can be applied, for example, it can be synthesized from xylylene diamine.

As an example, XDI can be synthesized from xylylene diamine through a phosgene method. For instance, the isocyanate may be produced by directly reacting xylylene diamine with phosgene in a solvent, or by forming its carbonate or hydrochloride, followed by reacting the amine salt with phosgene (COCl2) to synthesize XDI.

As another example, XDI can be synthesized from xylylene diamine through a non-phosgene method. For instance, biscarbamate can be produced by the reaction of xylylene diamine, urea and/or N-unsubstituted carbamic acid ester, and alcohol. The biscarbamate can be pyrolyzed or degassed, for example, in the presence of a catalyst to produce XDI.

In particular, the isocyanate composition of the present disclosure, which comprises the compound represented by Chemical Formula 1 in a content of greater than 60 ppm to 200 ppm or less, exhibits less cloudiness and discoloration even when stored for a long time of several months or more, for example, 12 months or more. Furthermore, poly(thio)urethane lenses and the like manufactured using such an isocyanate composition exhibit excellent transparency and can be suitably used as high-quality optical products.

To ensure this effect, the content of the compound represented by Chemical Formula 1 may satisfy a range of greater than 60 ppm, or 61 ppm or more, or 61.5 ppm or more, or 62 ppm or more, or 62.5 ppm or more, or 63 ppm or more, or 63.5 ppm or more, or 64 ppm or more, or 64.5 ppm or more, or 65 ppm or more, or 65.5 ppm or more, or 66 ppm or more, or 66.5 ppm or more, or 67 ppm or more, or 67.5 ppm or more, or 68 ppm or more, and 200 ppm or less, or 180 ppm or less, or 170 ppm or less, or 160 ppm or less, or 150 ppm or less, or 140 ppm or less, or 130 ppm or less, or 120 ppm or less, or 110 ppm or less, or 100 ppm or less.

If the content of the compound represented by Chemical Formula 1 in the isocyanate composition is 60 ppm or less, the long-term storage stability of the isocyanate composition deteriorates, and if it exceeds 200 ppm, there is a problem that the discoloration resistance of the poly(thio)urethane lens decreases.

Meanwhile, the isocyanate composition of the present disclosure comprises xylylene diisocyanate and the compound represented by Chemical Formula 1 as essential components, with xylylene diisocyanate as the main component and the compound represented by Chemical Formula 1 as an auxiliary component.

Preferably, the compound represented by Chemical Formula 1 may be, for example, one represented by the following Chemical Formula 1-1:

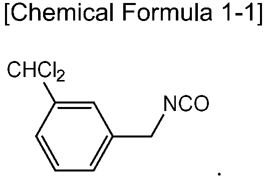

The compound represented by Chemical Formula 1 may be a commercially available product or may be prepared using one of various synthesis methods known in the art; the specific synthesis method is not particularly limited and can be applied. The specific preparation method is as shown in Preparation Example 1 to be described later.

Meanwhile, the compound represented by Chemical Formula 1 may be added to xylylene diisocyanate as a separate additive or may be added through a side reaction during the manufacturing process; as long as the aforementioned optimized content range can be satisfied, the specific generation method is not limited. In general, xylylene diisocyanate is prepared through a reaction of xylylene diamine or xylylene diamine hydrochloride or carbonate with phosgene and a purification process, and in some cases, the compound represented by Chemical Formula 1 is generated due to side reactions during such a reaction and/or purification process. Therefore, the content of the compound represented by Chemical Formula 1 can be adjusted to the aforementioned range by controlling the reaction conditions and/or purification conditions when preparing xylylene diisocyanate.

For example, the compound represented by Chemical Formula 1 is a chlorine compound produced as a by-product in the preparation of xylylene diisocyanate described later. The compound represented by Chemical Formula 1 (dichloromethylbenzyl isocyanate, DCMBI) may be at least one selected from the group consisting of structural isomers: 2-(dichloromethyl)benzyl isocyanate (o-(dichloromethyl)benzyl isocyanate, o-DCMBI), 3-(dichloromethyl)benzyl isocyanate (m-(dichloromethyl)benzyl isocyanate, m-DCMBI), and 4-(dichloromethyl)benzyl isocyanate (p-(dichloromethyl)benzyl isocyanate, p-DCMBI).

Thus, the structural isomer of dichloromethylbenzyl isocyanate produced as a by-product in the preparation of xylylene diisocyanate corresponds to the structural isomer of the manufactured xylylene diisocyanate. Therefore, the xylylene diisocyanate composition contains dichloromethylbenzyl isocyanate corresponding to the above-described structural isomer of xylylene diisocyanate. That is, if the xylylene diisocyanate composition contains 1,2-xylylene diisocyanate (o-xylylene diisocyanate, o-XDI), it contains 2-(dichloromethyl)benzyl isocyanate (o-(dichloromethyl)benzyl isocyanate, o-DCMBI); if it contains 1,3-xylylene diisocyanate (m-xylylene diisocyanate, m-XDI), it contains 3-(dichloromethyl)benzyl isocyanate (m-(dichloromethyl)benzyl isocyanate, m-DCMBI); and if it contains 1,4-xylylene diisocyanate (p-xylylene diisocyanate, p-XDI), it contains 4-(dichloromethyl)benzyl isocyanate (p-(dichloromethyl)benzyl isocyanate, p-DCMBI).

Preferably, the compound represented by Chemical Formula 1 (dichloromethylbenzyl isocyanate) may be 3-(dichloromethyl)benzyl isocyanate (m-(dichloromethyl)benzyl isocyanate, m-DCMBI).

Accordingly, in order to prepare the isocyanate composition according to an embodiment of the present disclosure, it is first necessary to analyze the content of the compound of Chemical Formula 1 in xylylene diisocyanate, which is the main raw material of the isocyanate composition. Thereafter, if necessary, the compound of Chemical Formula 1 can be further added, or the content of the compound of Chemical Formula 1 in xylylene diisocyanate can be adjusted through purification methods such as distillation or column chromatography, thereby obtaining the isocyanate composition of the present disclosure described above. The method for analyzing the content of the compound of Chemical Formula 1 in the isocyanate composition is not particularly limited, and for example, it can be measured by gas chromatography (GC-MS), etc.

For example, the isocyanate composition according to an embodiment of the present disclosure may be prepared by reacting xylylene diamine or a xylylene diamine salt (for example, hydrochloride or carbonate) with phosgene to obtain a reaction mixture, and purifying the reaction mixture under reduced pressure at a temperature of greater than 120 °C and less than 230 °C. For example, the purification step may be performed under reduced pressure conditions of 1 bar or less, or 0.98 bar or less, or 0.95 bar or less, or 0.9 bar or less, and in practical aspects, it may be performed under reduced pressure conditions of 0.01 mbar or more. In addition, the purification step may be performed under temperature conditions of 125 °C or more, or 130 °C or more, or 140 °C or more, and 225 °C or less, or 220 °C or less, or 210 °C or less, or 200 °C or less.

Here, the step of reacting the xylylene diamine or xylylene diamine salt with phosgene, that is, the phosgenation reaction, may be performed according to conventionally known methods. For example, xylylene diamine or its salt may be dispersed in at least one solvent selected from the group consisting of aromatic hydrocarbon solvents such as benzene, toluene, xylene, and ethylbenzene; chlorinated aromatic hydrocarbon solvents such as monochlorobenzene, 1,2-dichlorobenzene, and 1,4-dichlorobenzene; and chlorinated hydrocarbon solvents such as dichloromethane, chloroform, and carbon tetrachloride, and then phosgene may be injected, followed by performing the phosgenation reaction at a temperature of 100 °C or more, or 120 °C or more, and 150 °C or less, or 140 °C or less.

For example, the method for preparing the isocyanate composition comprises: reacting xylylene diamine or a xylylene diamine salt with phosgene to obtain a reaction mixture; and removing the gas phase from the reaction mixture. In addition, it may further comprise a desolvation step of removing the solvent and a purification step of removing impurities from the reaction product from which the gas phase has been removed. Specifically, the method includes a degassing step of removing the gas phase from the reaction mixture; and performing a desolvation step of removing the solvent and a purification step of removing impurities from the reaction product from which the gas phase has been removed, thereby preparing an isocyanate composition containing the compound represented by Chemical Formula 1. However, the method for preparing the isocyanate composition is not limited to the above, and an isocyanate composition in which the content of the compound represented by Chemical Formula 1 is greater than 60 ppm to 200 ppm or less can be prepared by controlling the conditions during the phosgenation reaction, purification conditions, and/or purification techniques.

In addition, the isocyanate composition according to an embodiment of the present disclosure may have a chlorine (Cl) content of 200 ppm or less, or greater than 0 to 200 ppm or less, based on the total weight of the isocyanate composition, measured using a combustion ion chromatograph in accordance with the American Society for Testing and Materials (ASTM) D 7359 method.

Specifically, the chlorine (Cl) content may be less than 200 ppm, or 190 ppm or less, or 180 ppm or less, or 170 ppm or less, or 160 ppm or less, or 150 ppm or less, or 145 ppm or less, or 140 ppm or less, or 135 ppm or less, or 132 ppm or less. However, in practical aspects, the chlorine (Cl) content may be 0.1 ppm or more, or 0.5 ppm or more, or 0.8 ppm or more, or 1 ppm or more, or 1.5 ppm or more, or 3 ppm or more, or 5 ppm or more, or 10 ppm or more, or 15 ppm or more, or 18 ppm or more, or 20 ppm or more, or 25 ppm or more, or 30 ppm or more, or 35 ppm or more, or 40 ppm or more, or 42 ppm or more, or 45 ppm or more, or 48 ppm or more, or 50 ppm or more, or 52 ppm or more, or 55 ppm or more, or 58 ppm or more, or 60 ppm or more, or 61 ppm or more. The chlorine (Cl) content is preferably 150 ppm or less, or 145 ppm or less, or 140 ppm or less, or 135 ppm or less, and in practical aspects, it may be 50 ppm or more, or 52 ppm or more, or 55 ppm or more, or 60 ppm or more.

In addition, the isocyanate composition according to an embodiment of the present disclosure may have an APHA color index of 0.1 or more to 25 or less, measured using a spectrophotometer (Ultrascan Pro by HunterLab, light source: C/2) in accordance with the American Society for Testing and Materials (ASTM) E313 method. Here, the isocyanate composition may have an APHA value of 0.1 or more and 10 or less immediately after preparation, and the APHA color index of the isocyanate composition may increase during storage. Specifically, the APHA color index of the isocyanate composition immediately after preparation may be 10 or less, or 9.5 or less, or 9 or less, or 8.5 or less, or 8 or less, and may be 0.1 or more, or 0.2 or more, or 0.5 or more, or 1 or more, or 1.5 or more, or 2 or more, or 2.5 or more, or 3 or more, or 3.5 or more, or 4 or more, or 4.5 or more, or 5 or more. Also, after 12 months of storage, the APHA color index of the isocyanate composition may be 25 or less, or 22 or less, or 20 or less, or 18 or less, and may be 0.5 or more, or 1 or more, or 3 or more, or 5 or more, or 8 or more, or 10 or more, or 12 or more, or 14 or more.

Meanwhile, the isocyanate composition according to an embodiment of the present disclosure may further include at least one additional component selected from the group consisting of chloromethylbenzyl isocyanate, isocyanomethylbenzaldehyde (IMBAI), and isocyanomethylbenzamide (IMBAm).

Specifically, the chloromethylbenzyl isocyanate may be represented by the following Chemical Formula 2, the isocyanomethylbenzaldehyde may be represented by the following Chemical Formula 3, and the isocyanomethylbenzamide (IMBAm) may be represented by the following Chemical Formula 4.

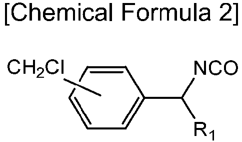

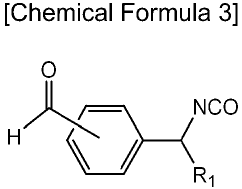

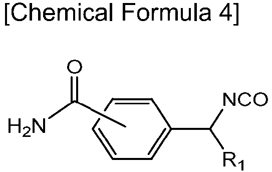

In Chemical Formulas 2 to 4,
R₁ is each independently hydrogen, methyl, or ethyl.

For example, the chloromethylbenzyl isocyanate may be represented by the following Chemical Formula 2-1, the isocyanomethylbenzaldehyde may be represented by the following Chemical Formula 3-1, and the isocyanomethylbenzamide may be represented by the following Chemical Formula 4-1.

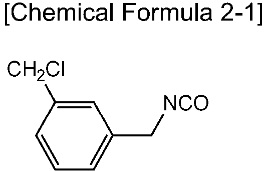

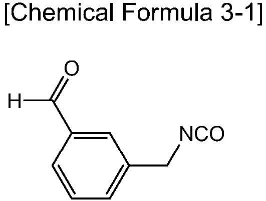

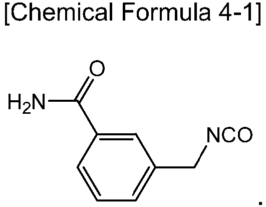

In the isocyanate composition according to an embodiment of the present disclosure, at least one additional component selected from the group consisting of chloromethylbenzyl isocyanate, isocyanomethylbenzaldehyde, and isocyanomethylbenzamide may be independently included in an amount of 0.5 parts by weight or less, or 0 or more and 0.5 parts by weight or less, per 100 parts by weight of the isocyanate composition.

In particular, in the isocyanate composition, the content of at least one additional component selected from the group consisting of chloromethylbenzyl isocyanate, isocyanomethylbenzaldehyde, and isocyanomethylbenzamide immediately after preparation may be each independently less than 0.4 parts by weight, or 0.38 parts by weight or less, or 0.35 parts by weight or less, or 0.32 parts by weight or less, or 0.3 parts by weight or less, or 0.28 parts by weight or less, or 0.25 parts by weight or less, or 0.22 parts by weight or less, or 0.2 parts by weight or less, or 0.18 parts by weight or less, or 0.15 parts by weight or less, or 0.12 parts by weight or less, or 0.1 parts by weight or less, or 0.09 parts by weight or less, or 0.05 parts by weight or less, or 0.03 parts by weight or less, or 0.01 parts by weight or less, and 0.001 parts by weight or more, 0.002 parts by weight or more, 0.003 parts by weight or more, or 0.005 parts by weight or more, or 0.007 parts by weight or more.

Meanwhile, the isocyanate composition according to an embodiment of the present disclosure can optimize the content of the compound (DCMBI) represented by Chemical Formula 1, thereby lowering the rate at which the content of at least one additional component selected from the group consisting of isocyanomethylbenzaldehyde (IMBAl) and isocyanomethylbenzamide (IMBAm) increases after long-term storage for 12 months. For example, at least one additional component selected from the group consisting of isocyanomethylbenzaldehyde and isocyanomethylbenzamide in the isocyanate composition stored for 12 months may be each independently included in an amount of 0.01 parts by weight or more and 0.5 parts by weight or less per 100 parts by weight of the isocyanate composition. Specifically, as described above, at least one additional component among IMBAl and IMBAm in the XDI composition after 12 months may be each independently less than 0.4 parts by weight, or 0.38 parts by weight or less, or 0.35 parts by weight or less, or 0.32 parts by weight or less, or 0.3 parts by weight or less, or 0.28 parts by weight or less, or 0.25 parts by weight or less, or 0.22 parts by weight or less, or 0.2 parts by weight or less, or 0.18 parts by weight or less, or 0.15 parts by weight or less, or 0.12 parts by weight or less, or 0.1 parts by weight or less, or 0.09 parts by weight or less, and 0.02 parts by weight or more, or 0.03 parts by weight or more, or 0.04 parts by weight or more, or 0.05 parts by weight or more, or 0.06 parts by weight or more.

Here, the content of at least one selected from the group consisting of chloromethylbenzyl isocyanate, isocyanomethylbenzaldehyde, and isocyanomethylbenzamide can be measured through component analysis by gas chromatography (GC), but is not limited thereto, and may be measured by other methods known in the art. For example, the method for measuring the content of such additional components is as described in Test Example 3 below.

Specifically, the content of at least one selected from the group consisting of chloromethylbenzyl isocyanate, isocyanomethylbenzaldehyde, and isocyanomethylbenzamide can be measured by performing gas chromatography (GC) analysis under the following analysis conditions.

### 1. GC Measurement Conditions

Equipment: GC (Agilent HP-7890A FID)
Column: DB-17 (30m x 0.25mm, 0.5µm)
Split ratio: 100:1
Inlet temperature: 250 °C
Detector temperature: 280 °C
Oven temperature: 80 °C → 5 °C/min ramp up, 160 °C (8 min hold) → 20 °C/min ramp up, 280 °C (18 min hold)
Carrier gas: N₂
Carrier gas flow rate: 1.0 ml/min (constant flow control)
Sample concentration: 1.0 wt% dichloromethane solution
Injection volume: 1.0 µL

When gas chromatography (GC) analysis is performed under the above conditions, the content ratio of each compound relative to the total weight of the isocyanate composition can be expressed as the content (wt%) of each component.

For example, according to the gas chromatography (GC) analysis described above, the retention time of xylylene diisocyanate (XDI) represents a compound within 27.9 minutes to 29.0 minutes, isocyanomethylbenzaldehyde (IMBAl) represents a compound with a retention time of 24.7 minutes to 26.4 minutes, and isocyanomethylbenzamide (IMBAm) represents a compound with a retention time of 30.7 minutes to 32.8 minutes.

In particular, by including one or more of the aforementioned isocyanomethylbenzaldehyde and isocyanomethylbenzamide together with the compound represented by Chemical Formula 1 (dichloromethylbenzyl isocyanate, DCMBI), they play a role in suppressing the generation of uretdione, which is a self-polymerized compound generated during long-term storage of several months or more, and additional impurities, thereby reducing the increase range of the APHA of the composition and the YI of the lens. For reference, the content of chloromethylbenzyl isocyanate does not change significantly during storage.

The isocyanate composition according to an embodiment of the present disclosure may have a uretdione content measured after 12 months of long-term storage, that is, the uretdione content in the XDI composition after 12 months of storage (content of uretdione in the XDI composition after 12 months), of 5 parts by weight or less per 100 parts by weight of the isocyanate composition.

Here, uretdione is a form in which two NCO groups, each of two xylylene diisocyanates (XDI), are bonded to each other in a cyclic form, which is referred to as uretdione, and two XDI molecules can be bonded into one ring. In addition, three XDI molecules can be generated in a form having two uretdione rings. At this time, the compound represented by Chemical Formula 1 (dichloromethylbenzyl isocyanate, DCMBI) may be generated by the bonding of NCOs of various substances in the isocyanate composition, such as the bonding of NCO of XDI.

Specifically, in the present disclosure, uretdione may be represented by the following Chemical Formula 5.

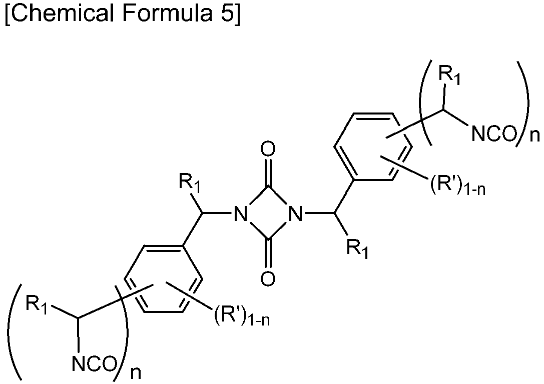

In Chemical Formula 5,
R₁ is each independently hydrogen, methyl, or ethyl,
R' is each independently a chloromethyl group (-CH₂Cl), a dichloromethyl group (-CHCl₂), an aldehyde group (-CHO), or an amide group (-CONH₂), and
n is each independently 0 or 1.

For example, the uretdione may be represented by any one of the following Chemical Formulas 5-1 to 5-3.

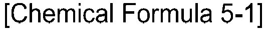

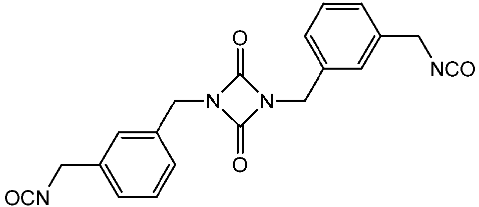

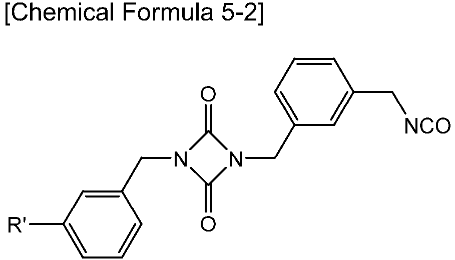

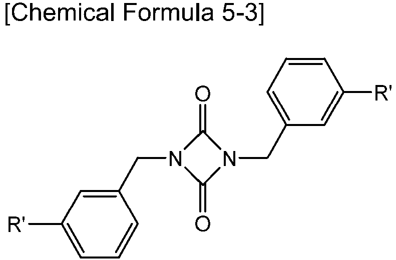

In Chemical Formulas 5-1 to 5-3,
R' is as defined above in Chemical Formula 5.

In particular, the content of the uretdione, that is, the content of uretdione measured after the isocyanate composition has been stored for a long period of 12 months (content of uretdione in the XDI composition after 12 months), may be 5 parts by weight or less, or 0 to 5 parts by weight or less, based on 100 parts by weight of the isocyanate composition. The content of uretdione after 12 months of storage is preferably less than 5 parts by weight, or 4.9 parts by weight or less, or 4.8 parts by weight or less, or 4.5 parts by weight or less, or 4.2 parts by weight or less, or 4 parts by weight or less, or 3.8 parts by weight or less, or 3.5 parts by weight or less, or 3.2 parts by weight or less, or 3 parts by weight or less, or 2.85 parts by weight or less, or 2.7 parts by weight or less. However, in terms of the actual self-polymerization process that occurs during long-term storage, the content of uretdione after 12 months of storage may be greater than 0 parts by weight, or 0.01 parts by weight or more, or 0.02 parts by weight or more, or 0.03 parts by weight or more, or 0.04 parts by weight or more, or 0.05 parts by weight or more, or 0.06 parts by weight or more, or 0.08 parts by weight or more, or 0.1 parts by weight or more.

Here, the content of uretdione can be measured through infrared spectroscopy (IR) analysis, but is not limited thereto, and may be measured by other methods known in the art. For example, the method for measuring the content of such uretdione is as described in Test Example 3 below.

Specifically, after storing the isocyanate composition of the present disclosure in a general tin can under room temperature (about 15 °C to 25 °C) conditions for 12 months, the content of uretdione in the thus-obtained isocyanate (XDI) composition after 12 months of storage can be measured through analysis by infrared spectroscopy (IR). For example, after obtaining an infrared absorption spectrum for the isocyanate composition after 12 months of storage using the ATR mode of an FT-IR spectrometer (Varian 4100), the area ratio of the absorption peak of the uretdione carbonyl group (1770-1790 cm⁻¹) is measured, and based on the absorption coefficient obtained from the calibration curve, the uretdione content (wt%) is calculated.

When infrared spectroscopy (IR) analysis is performed under the above-described conditions, the weight ratio of the uretdione compound relative to the total weight of the isocyanate composition can be expressed as the content (wt%).

Meanwhile, the isocyanate composition according to an embodiment of the present disclosure may satisfy the aforementioned chlorine (Cl) content range measured in the initial stage after preparation after being stored for a long period of 12 months.

### Polymerizable Composition

According to another embodiment of the present disclosure, there is provided a polymerizable composition for polyisocyanate polymerization, comprising the above-described isocyanate composition and at least one polyfunctional monomer compound selected from the group consisting of a polyfunctional alcohol-based compound, a polyfunctional thiol-based compound, and a polyfunctional episulfide-based compound.

The polymerizable composition uses an isocyanate composition that contains xylylene diisocyanate and the compound represented by Chemical Formula 1, with a content satisfying greater than 60 ppm to 200 ppm or less, thereby facilitating the control of reactivity during polymerization.

The polymerizable composition has high transparency and excellent impact strength, making it suitable for use as a high-quality optical material.

The description of the isocyanate composition included in the polymerizable composition is the same as described above.

Preferably, the polymerization composition may contain the isocyanate composition and the polyfunctional monomer compound in a mixed state, or they may be contained in a state separated from each other. That is, in the polymerization composition, the isocyanate composition and the polyfunctional monomer compound may be in a state of being mixed in contact with each other, or in a state of being separated so as not to come into contact with each other.

The polyfunctional alcohol-based compound is a compound containing two or more hydroxyl groups (-OH) per molecule, specifically, a compound having 2 or more, or 3 or more, and 8 or less, or 4 or less hydroxyl groups in the molecule. Specific examples include dihydric alcohols such as ethylene glycol, diethylene glycol, triethylene glycol, 1,2-propanediol, 1,3-propanediol, 1,2-butanediol, 2-methyl-2,3-butanediol, 1,6-hexanediol, 1,2-hexanediol, polypropylene glycol, polytetramethylene ether glycol, ethylene oxide-propylene oxide block copolymer, polyalkylene carbonate diol, polycaprolactone diol; trihydric alcohols such as glycerol, trimethylolethane, trimethylolpropane (TMP); tetrahydric alcohols such as diglycerin, ditrimethylolpropane, pentaerythritol, dipentaerythritol; pentahydric alcohols such as L-arabinitol, ribitol, xylitol; hexahydric alcohols such as D-glucitol, D-mannitol, galactitol; heptahydric alcohols such as trehalose; octahydric alcohols such as sucrose, maltose; or polyols, and any one or a mixture of two or more thereof can be used.

The polyfunctional thiol-based compound is a compound containing two or more thiol groups (-SH) per molecule, specifically, a compound having 2 or more, or 3 or more, and 8 or less, or 5 or less thiol groups in the molecule.

The polyfunctional thiol-based compound may be, for example, 2,3-bis(2-sulfanylethylsulfanyl)propane-1-thiol, 1,9-dimercapto-3,7-dithianonane, 1,13-dimercapto-3,7,11-trithiatridecane, glycol di(3-mercaptopropionate), 1,4-dithiane-2,5-diyldimethanethiol, 2-mercaptomethyl-1,5-dimercapto-3-thiapentane, trimethylolpropane tri(3-mercaptopropionate), 4,8-di(mercaptomethyl)-1,11-dimercapto-3,6,9-trithiaundecane, 5,9-di(mercaptoethyl)-1,12-dimercapto-3,7,10-trithiadodecane, pentaerythritol tetra(3-mercaptopropionate), pentaerythritol tetra(mercaptoacetate), 3,6,9,12-tetrathiatetradecane-1,14-dithiol, 3,6,10,13-tetrathiapentadecane-1,8,15-trithiol.

Specifically, the polyvalent thiol can be added in an amount such that the molar ratio of the thiol group in the polyvalent thiol to 1 mole of the isocyanate group of the isocyanate compound is 0.8 or more, or 0.9 or more, and 1.1 or less, or 1.0. If the molar ratio of the thiol group to the isocyanate group is outside the above range and less than 0.8, the viscosity of the produced polymer is lowered due to the excess isocyanate groups, which may cause a decrease in workability. Also, if the molar ratio of the thiol group to the isocyanate group exceeds 1.1, there is a concern that the discoloration prevention effect may be reduced due to the excess thiol groups.

The polyfunctional episulfide-based compound may be a compound containing two or more episulfides, i.e., thioepoxy groups, in the molecule, and may have an aliphatic, alicyclic, or aromatic backbone. According to an example, the polyfunctional episulfide-based compound may be bis(β-epithiopropylthio)methane, 1,2-bis(β-epithiopropylthio)ethane, 1,3-bis(β-epithiopropylthio)propane, 1,2-bis(β-epithiopropylthio)propane, 1-(β-epithiopropylthio)-2-(β-epithiopropylthiomethyl)propane, 1,4-bis(β-epithiopropylthio)butane, 1,3-bis(β-epithiopropylthio)butane, 1-(β-epithiopropylthio)-3-(β-epithiopropylthiomethyl)butane, 1,5-bis(β-epithiopropylthio)pentane, 1-(β-epithiopropylthio)-4-(β-epithiopropylthiomethyl)pentane, 1,6-bis(β-epithiopropylthio)hexane, 1-(β-epithiopropylthio)-5-(β-epithiopropylthiomethyl)hexane, 1-(β-epithiopropylthio)-2-[(2-β-epithiopropylthioethyl)thio]ethane, 1-(β-epithiopropylthio)-2-[[2-(2-β-epithiopropylthioethyl)thioethyl]thio]ethane, tetrakis(β-epithiopropylthiomethyl)methane, 1,1,1-tris(β-epithiopropylthiomethyl)propane, 1,5-bis(β-epithiopropylthio)-2-(β-epithiopropylthiomethyl)-3-thiapentane, 1,5-bis(β-epithiopropylthio)-2,4-bis(β-epithiopropylthiomethyl)-3-thiapentane, 1-(β-epithiopropylthio)-2,2-bis(β-epithiopropylthiomethyl)-4-thiahexane, 1,5,6-tris(β-epithiopropylthio)-4-(β-epithiopropylthiomethyl)-3-thiahexane, 1,8-bis(β-epithiopropylthio)-4-(β-epithiopropylthiomethyl)-3,6-dithiaoctane, 1,8-bis(β-epithiopropylthio)-4,5-bis(β-epithiopropylthiomethyl)-3,6-dithiaoctane, 1,8-bis(β-epithiopropylthio)-4,4-bis(β-epithiopropylthiomethyl)-3,6-dithiaoctane, 1,8-bis(β-epithiopropylthio)-2,4,5-tris(β-epithiopropylthiomethyl)-3,6-dithiaoctane, 1,8-bis(β-epithiopropylthio)-2,5-bis(β-epithiopropylthiomethyl)-3,6-dithiaoctane, 1,9-bis(β-epithiopropylthio)-5-(β-epithiopropylthiomethyl)-5-[(2-β-epithiopropylthioethyl)thiomethyl]-3,7-dithianonane, 1,10-bis(β-epithiopropylthio)-5,6-bis[(2-β-epithiopropylthioethyl)thio]-3,6,9-trithiadecane, 1,11-bis(β-epithiopropylthio)-4,8-bis(β-epithiopropylthiomethyl)-3,6,9-trithiaundecane, 1,11-bis(β-epithiopropylthio)-5,7-bis(β-epithiopropylthiomethyl)-3,6,9-trithiaundecane, 1,11-bis(β-epithiopropylthio)-5,7-[(2-β-epithiopropylthioethyl)thiomethyl]-3,6,9-trithiaundecane, 1,11-bis(β-epithiopropylthio)-4,7-bis(β-epithiopropylthiomethyl)-3,6,9-trithiaundecane, 1,3-bis(β-epithiopropylthio)cyclohexane, 1,4-bis(β-epithiopropylthio)cyclohexane, 1,3-bis(β-epithiopropylthiomethyl)cyclohexane, 1,4-bis(β-epithiopropylthiomethyl)cyclohexane, bis[4-(β-epithiopropylthio)cyclohexyl]methane, 2,2-bis[4-(β-epithiopropylthio)cyclohexyl]propane, bis[4-(β-epithiopropylthio)cyclohexyl]sulfide, 2,5-bis(β-epithiopropylthiomethyl)-1,4-dithiane, 2,5-bis(β-epithiopropylthioethylthiomethyl)-1,4-dithiane, 1,3-bis(β-epithiopropylthio)benzene, 1,4-bis(β-epithiopropylthio)benzene, 1,3-bis(β-epithiopropylthiomethyl)benzene, 1,4-bis(β-epithiopropylthiomethyl)benzene, bis[4-(β-epithiopropylthio)phenyl]methane, 2,2-bis[4-(β-epithiopropylthio)phenyl]propane, bis[4-(β-epithiopropylthio)phenyl]sulfide, bis[4-(β-epithiopropylthio)phenyl]sulfone, and 4,4'-bis(β-epithiopropylthio)biphenyl.

The polymerization composition may further include additives such as an internal release agent, an ultraviolet absorber, a urethane reaction catalyst, a polymerization initiator, a heat stabilizer, a color corrector, a chain extender, a crosslinking agent, a light stabilizer, a filler, a photosensitizer, etc., as needed, within a range that does not degrade the effects desired by the present invention, and the content can be appropriately determined within a range that does not inhibit the discoloration and discoloration suppression characteristics of the composition.

The polymerization composition described above can control the occurrence of cloudiness by delaying or inhibiting the reaction rate and oligomerization of the isocyanate due to the combination of the two types of antioxidants contained in the isocyanate composition.

The polyol contained in the polymerizable composition is a compound having 2 or more hydroxy groups, and compounds commonly used in polyurethane production can be used without limitation. For example, the polyol includes dihydric alcohols such as ethylene glycol, diethylene glycol, triethylene glycol, 1,2-propanediol, 1,3-propanediol, 1,2-butanediol, 2-methyl-2,3-butanediol, 1,6-hexanediol, 1,2-hexanediol; trihydric alcohols such as glycerol, trimethylolethane, trimethylolpropane (TMP); tetrahydric alcohols such as diglycerin, ditrimethylolpropane, pentaerythritol, dipentaerythritol; pentahydric alcohols such as L-arabinitol, ribitol, xylitol; hexahydric alcohols such as D-glucitol, D-mannitol, galactitol; heptahydric alcohols such as trehalose; octahydric alcohols such as sucrose, maltose; or low molecular weight polyols such as polyalkylene oxide with a number average molecular weight of 60 to 400.

Among them, diethylene glycol, glycerol, trimethylolethane, trimethylolpropane, or mixtures thereof can be used, and more specifically, it may be preferable to use a trihydric alcohol such as glycerol, trimethylolpropane, or trimethylolethane alone, or to use a mixture of the trihydric alcohol and other polyols.

The polythiol is a compound having 2 or more mercapto groups, and compounds used in polythiourethane production can be used without limitation. For example, aliphatic polythiols such as 2,3-bis(2-sulfanylethylsulfanyl)propane-1-thiol, 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane can be used alone or in combination of two or more, but the present invention is not limited thereto.

The polymerizable composition may further include a polymerization initiator, an internal release agent, and an ultraviolet absorber, as needed.

As the polymerization initiator, amine-based, phosphorus-based, organotin-based, organocopper-based, organogallium, organozirconium, organoiron-based, organozinc, organoaluminum, etc. can be used. The polymerization initiator may be included in an amount of 0.001 to 0.1 parts by weight based on 100 parts by weight of the polymerizable composition.

As the internal release agent, a fluorine-based nonionic surfactant having a perfluoroalkyl group, hydroxyalkyl group, or phosphoric acid ester group; a silicone-based nonionic surfactant having a dimethylpolysiloxane group, hydroxyalkyl group, or phosphoric acid ester group; an alkyl quaternary ammonium salt, i.e., trimethylcetyl ammonium salt, trimethylstearyl, dimethylethylcetyl ammonium salt, triethyldodecyl ammonium salt, trioctylmethyl ammonium salt, diethylcyclohexadodecyl ammonium salt; or a phosphoric acid ester-based compound such as acidic phosphoric acid ester may be selected and used alone or in combination of two or more. Preferably, a phosphoric acid ester-based compound can be used. The internal release agent may be used in an amount of 0.01 to 1 part by weight per 100 parts by weight of the polymerizable composition.

As the ultraviolet absorber, for example, benzotriazole compounds (specifically, Tinuvin 571, Tinuvin 213, Tinuvin 234, Tinuvin P (above, manufactured by BASF)), formamidine-based compounds (specifically, Zikasorb R, Zikasorb BS, ZIKA-FA02, ZIKA-FUA, ZIKA-FUV, ZIKA-UVS3, ZIKA-UVS4 (above, manufactured by ZIKO)), Biosorb 583 (manufactured by Sakai Chemical Industry Co., Ltd.), etc., can be mentioned. The ultraviolet absorber may be used in an amount of 0.01 to 1 part by weight per 100 parts by weight of the polymerizable composition.

The polymerization composition described above contains the compound represented by Chemical Formula 1 as described above in the isocyanate composition, and by optimizing its content to be greater than 60 ppm to 200 ppm or less, the reaction rate and oligomerization of the isocyanate are delayed or inhibited, thereby controlling the occurrence of cloudiness and discoloration even during long-term storage of several months or more, for example, 12 months or more, and at the same time, it can improve the transparency of the lens when used as a raw material for optical articles such as poly(thio)urethane lenses.

### Polyisocyanate Composition

According to another embodiment of the present disclosure, there is provided a polyisocyanate composition comprising a polyisocyanate obtained by polymerizing the above-described isocyanate composition and a polyfunctional alcohol-based compound. Polyisocyanate manufactured by reaction with a polyhydric alcohol in this way exhibits excellent transparency as well as excellent pressure-sensitive/adhesive properties. Therefore, it can be used for coatings, pressure-sensitive adhesives, adhesives, etc., and is not limited thereto, and is useful for manufacturing optical pressure-sensitive adhesives, optical adhesives, optical coatings, or optical coatings.

Here, the description of the polyfunctional alcohol-based compound can be applied in the same manner as the above description.

According to another embodiment of the present disclosure, there is provided a polyisocyanate composition comprising a polyisocyanate obtained by polymerizing the above-described isocyanate composition and a polyfunctional thiol-based compound or a polyfunctional episulfide-based compound. The polyisocyanate composition can be used for optical materials, for example, optical lenses, but is not limited thereto.

Here, the description of the polyfunctional thiol-based compound and the polyfunctional episulfide-based compound can be applied in the same manner as the above description.

In the polyisocyanate composition, the polymerization reaction between the isocyanate composition and the monomer compound (polyfunctional alcohol-based compound, polyfunctional thiol-based compound, polyfunctional episulfide-based compound) may be performed under normal pressure conditions and in an inert gas atmosphere such as nitrogen or argon.

The polymerization reaction may be performed in a temperature range of - 15 °C or more, or 0 °C or more, and 200 °C or less, or 170 °C or less. For example, when a polyfunctional alcohol-based compound is used as the monomer compound, it is preferably performed at 60 to 80 °C, and when a polyfunctional thiol-based compound or a polyfunctional episulfide-based compound is used as the monomer compound, it is preferably performed at 100 °C to 160 °C.

When the polymerization reaction is performed in the above temperature range, the reaction rate can be easily controlled without concern for discoloration, and the reaction efficiency can also be increased, which is preferable.

The polymerization reaction may be performed under catalyst-free conditions or in the presence of a catalyst that promotes the urethanization reaction, such as a tin-based or amine-based catalyst. When performed in the presence of a catalyst, the catalyst may be added together during the step of adding the monomer compound to the isocyanate-based mixture.

When a polyfunctional alcohol-based compound is used in the polymerization reaction, the progress of the reaction can be predicted by measuring the concentration of isocyanate groups in the polymerization reaction product by the n-dibutyl amine method using a potentiometric titration device, or by measuring the refractive index, and in the present disclosure, the reaction can be performed until the concentration of isocyanate groups in the polymerization reaction product reaches the calculated value of the isocyanate groups remaining after reacting with the monomer compound. As a result of the polymerization reaction as described above, polyisocyanate is produced.

The polyisocyanate composition may further include additives such as an internal release agent, an ultraviolet absorber, a polymerization initiator, a heat stabilizer, an antioxidant, a color corrector, a chain extender, a crosslinking agent, a light stabilizer, a filler, a photosensitizer, etc., as needed, within a range that does not degrade the effects desired by the present invention, and the content can be appropriately determined within a range that does not inhibit the discoloration and discoloration suppression characteristics of the composition.

### Optical Article

According to another embodiment of the present disclosure, there is provided an article comprising a polymer obtained by polymerizing the above-described isocyanate composition and at least one polyfunctional monomer compound selected from the group consisting of a polyfunctional thiol-based compound, a polyfunctional alcohol-based compound, and a polyfunctional episulfide-based compound.

This may also be an article comprising a polymer obtained by polymerizing an isocyanate composition and a polyfunctional monomer compound in the polymerization composition described above.

The polyisocyanate composition exhibits excellent transparency and excellent pressure-sensitive/adhesive properties, and thus can be applied to a wide range of fields. Specifically, it can be variously utilized as an optical material as well as a coating, a pressure-sensitive adhesive, an adhesive, and more preferably, the article can be used as an optical pressure-sensitive adhesive, an optical adhesive, an optical coating, or an optical lens.

The article may be manufactured by performing a molding process after the polymerization reaction in the above-described composition, or may be manufactured through a molding process using the polymerization composition. In the latter case, the polymerization reaction occurs simultaneously during the molding process.

Preferably, when used as an optical lens, it may be a polymer obtained by polymerizing the isocyanate composition and a polyfunctional thiol-based compound or a polyfunctional episulfide-based compound, in which case the polymerization composition containing the isocyanate composition and the polyfunctional thiol-based compound or the polyfunctional episulfide-based compound is injected into a lens molding mold, and then the temperature of the mold is raised to perform the polymerization reaction between the isocyanate-based compound and the polyfunctional monomer compound. Here, the mold is heated to a temperature range where the urethane polymerization reaction occurs, and after the polymerization reaction is completed, the produced polymer, specifically polythiourethane, is separated from the mold to obtain an optical lens.

For example, an optical lens according to another embodiment of the present disclosure may have an Abbe number (Ve) of 25 or more, or 28 or more, or 30 or more, or 31 or more, or 31.4 or more, and in practical aspects, it may be 50 or less, or 45 or less, or 43 or less, or 40 or less, or 38 or less, or 35 or less, or 32 or less. The method for measuring such an Abbe number (Ve) is as described in Test Example 2 described later, and specific description is omitted.

In addition, the optical lens may have a weather resistance index ΔYI, which is the deviation when comparing Y.I values before and after exposure to Xenon arc light source (irradiance 0.51W/m2@340nm, humidity 50%, temperature 60 °C) acceleration conditions for 110 hours, of 0.55 or less, or greater than 0 and 0.55 or less. The weather resistance index ΔYI of the lens may preferably be 0.54 or less, or 0.53 or less, and in some cases, 0.01 or more, or 0.05 or more, or 0.1 or more, or 0.2 or more, or 0.3 or more, or 0.4 or more, or 0.45 or more, or 0.47 or more.

The yellowness (YI, Yellowness) of the lens can be measured using a spectrophotometer (Ultrascan Pro by HunterLab) in accordance with the method of the American Society for Testing and Materials (ASTM) D1209.

In addition, when the optical lens is observed with the naked eye using a Xenon lamp light source device, no striae of the lens appear.

Accordingly, the optical lens can maintain excellent transparency while minimizing striae and discoloration due to non-uniform polymerization.

Meanwhile, an optical lens according to another embodiment of the present disclosure has a yellowness (YI) significantly lowered to 5 or less even when manufactured using an isocyanate composition stored for a long period of 12 months as described above in relation to the isocyanate composition, and there is no fear of discoloration or cloudiness even during long-term storage, and it can maintain excellent discoloration resistance and improve transparency. Preferably, the yellowness (YI) of the lens can be 4.8 or less, or 4.6 or less, or 4.4 or less, or 4.3 or less, even when manufactured using an isocyanate composition stored for a long period of 12 months, and in some cases, 0.01 or more, or 0.1 or more, or 0.5 or more, or 1 or more, or 1.5 or more, or 3 or more, or 3.5 or more, or 3.8 or more, or 4 or more.

Here, the method for measuring the yellowness (YI), weather resistance index ΔYI, and striae of the optical lens described above is as described in Test Example 2 described later, and specific description is omitted.

An optical element including a polymer obtained by polymerizing the polymerizable composition containing the isocyanate composition of the present disclosure described above has high transparency and exhibits improved impact resistance. Accordingly, the optical element can be usefully used as eyeglass lenses, camera lenses, adhesives requiring transparency, resins, etc.

Hereinafter, the present disclosure will be described in more detail with reference to the following examples. However, the following examples are only for illustrating the present disclosure, and the scope of the present disclosure is not limited thereto.

### [Examples]

### Preparation Example 1: Preparation of 3-dichloromethylbenzyl isocyanate (DCMBI)

To a mixed solution of 10 g (76.3 mmol) of 3-cyanobenzaldehyde and 500 mL of dichloromethane (CH₂Cl₂), 76 g (191.6 mmol) of tungsten chloride was added, and after reacting for 4 hours under reflux of dichloromethane, it was cooled to room temperature. Then, 200 mL of an aqueous sodium bicarbonate solution and 1100 mL of a 1 N aqueous sodium hydroxide solution were slowly added to the reaction solution in order while maintaining the temperature at 10 °C or less. Then, after separating the organic solvent layer, the dichloromethane layer, the water layer was extracted twice more with 500 mL of dichloromethane. The dichloromethane layer obtained by such separation and the dichloromethane layer obtained by the extraction process were mixed, and the dichloromethane layer was washed with 800 mL of brine and dried over magnesium sulfate. After drying in this way, magnesium sulfate was filtered off from the dichloromethane layer, and dichloromethane was distilled off to obtain a concentrated solution. Then, 13.2 g of 3-(dichloromethyl)benzonitrile was obtained from the concentrated solution by silica gel column chromatography.

Next, to a mixed solution of 10 g (53.8 mmol) of 3-(dichloromethyl)benzonitrile and 150 mL of tetrahydrofuran, 135 mL of a tetrahydrofuran solution (2M in THF) of borane-dimethyl sulfide complex was added dropwise at room temperature, followed by stirring for 24 hours to react. After reacting in this way, the reaction solution was immersed in ice water to lower the temperature, and 35 mL of a 2 M aqueous hydrochloric acid solution was slowly added. Then, 300 mL of ethyl acetate was added to the reaction solution and stirred for 10 minutes. Then, after separating and removing the ethyl acetate layer, it was again immersed in ice water, and 85 mL of 1 M sodium hydroxide was slowly added to the reaction solution. Thereafter, the reaction solution was extracted 4 times with 250 mL of dichloromethane, and the obtained dichloromethane layer was dried over magnesium sulfate.

After drying in this way, magnesium sulfate was filtered off from the dichloromethane layer, and dichloromethane was distilled off to obtain 5.5 g of 3-(dichloromethyl)benzylamine.

Next, to a mixed solution of 15.1 g (50.9 mmol) of triphosgene and 90 mL of o-dichlorobenzene, a mixed solution of 5.5 g (28.9 mmol) of the 3-(dichloromethyl)benzylamine obtained above and 90 mL of o-dichlorobenzene was added dropwise, and then reacted at 120 °C for 3 hours. After the reaction, it was cooled to room temperature, and o-dichlorobenzene was distilled off to obtain a concentrated solution. Then, 3.3 g of 3-(dichloromethyl)benzyl isocyanate (DCMBI) was finally obtained from the concentrated solution through silica gel column chromatography.

### Examples 1 to 3: Preparation of isocyanate composition

After measuring the 3-(dichloromethyl)benzyl isocyanate (DCMBI) content of meta-xylene diisocyanate (manufactured by TCI) through GC analysis as shown in Test Example 1 below, 3-(dichloromethyl)benzyl isocyanate (DCMBI) was added to each to reach the DCMBI content value in Table 1 below, thereby preparing the isocyanate compositions of Examples 1 to 3, respectively.

### Comparative Examples 1 to 3: Preparation of isocyanate composition

After measuring the 3-(dichloromethyl)benzyl isocyanate (DCMBI) content of meta-xylene diisocyanate (manufactured by TCI) through GC analysis as shown in Test Example 1 below, 3-(dichloromethyl)benzyl isocyanate (DCMBI) was added to each to reach the DCMBI content value in Table 1 below, thereby preparing the isocyanate compositions of Comparative Examples 1 to 3, respectively.

### [Test Example 1]

For the isocyanate compositions of Examples and Comparative Examples, the content of 3-(dichloromethyl)benzyl isocyanate (DCMBI) was measured by the following method, and the measured values are shown in Table 1 below.

### <DCMBI content measurement method>

Using DCMBI with a purity of 99 mol% synthesized according to Preparation Example 1 as a standard substance, it was analyzed by gas chromatography under the following conditions, and a calibration curve was prepared by adding the standard substance to the XDI sample. (Standard addition method)

Next, the XDI compositions of the above Examples and Comparative Examples were analyzed by gas chromatography under the following conditions to obtain the number of moles of DCMBI. This was converted into mass, and the content ratio of DCMBI in the XDI compositions of each Example and Comparative Example to be described later was calculated.

### 1. GC Measurement Conditions

Equipment: GC-MS (Agilent 8890 5977C)
Column: DB-17MS (30m x 0.25mm, 0.25µm)
Split ratio: 3:1
Inlet temperature: 200 °C
Detector temperature: 280 °C
Oven temperature: 50 °C (3 min hold) → 10 °C/min, 280 °C (6 min hold)
Carrier gas: He
Carrier gas flow rate: 1.0 ml/min (constant flow control)
Sample concentration: 1.0 mass% dichloromethane solution
Injection volume: 1.0 µL
Detection method: SIM mode (monitoring ions: m/z 180, 215)

**[Table 1]**

| | DCMBI content (ppm) |
|---|---|
| Example 1 | 68 |
| Example 2 | 100 |
| Example 3 | 150 |
| Comparative Example 1 | 5 |
| Comparative Example 2 | 40 |
| Comparative Example 3 | 330 |

### Preparation Example 2: Preparation of XDI adduct

Under a nitrogen atmosphere, 300 g of each m-XDI composition of Examples and Comparative Examples was added to a round-bottom flask and stirred. After raising the temperature of the flask to 70 °C and maintaining it, 26.7 g of trimethylolpropane (TMP) was added dropwise. After the completion of the addition, the reaction temperature was maintained while maintaining 70 °C until the isocyanate group concentration reached the calculated value of 33%.

After the completion of the reaction, the resulting reaction product was purified using a thin film evaporator (TFE) to separate unreacted XDI, thereby obtaining an XDI-TMP adduct.

The obtained XDI-TMP adduct was used after being diluted with ethyl acetate (solid content 75 wt%).

The color of the XDI adduct thus obtained was measured by sensory evaluation, and the results are shown in Table 2 below.

### Preparation Example 3: Preparation of optical lens

62.4 g of the isocyanate compositions prepared in Examples and Comparative Examples, 0.16 g of ZELEC^{™} UN (manufactured by Stepan) as an internal release agent, and 0.80 g of Tinuvin^{®} 329 (manufactured by BASF) as an ultraviolet absorber were stirred and mixed at room temperature (23 °C) for 20 minutes to prepare a mixture. 0.024 g of DBTC (dibutyltin dichloride) was added to the prepared mixture and stirred for 10 minutes, and 57.6 g of 2,3-bis(2-sulfanyl ethyl sulfanyl)propane-1-thiol was added, followed by stirring for 1 hour while degassing under a pressure condition of 5 mbar to prepare a composition for polyisocyanate polymerization.

The prepared composition for polyisocyanate polymerization was filtered using a 1 µm PTFE filter, and then injected into a mold type consisting of a glass mold and tape, respectively. Thereafter, the mold type was placed in an oven, and the polymerization reaction was performed for 20 hours while slowly raising the temperature from 10 °C to 120 °C. After the completion of the polymerization, the mold type was taken out of the oven and released to obtain each plastic optical lens. The obtained optical lenses were annealed at 120 °C for 6 hours to manufacture optical lenses each having a thickness of 9 mm.

### [Test Example 2]

For the isocyanate compositions of Examples and Comparative Examples, the XDI adducts manufactured using them, and the optical lenses, property evaluation was performed in the initial stage within 5 days after each preparation as follows, and the measurement results are shown in Table 2 below.

### <Measurement for Cl content of XDI composition>

In accordance with the American Society for Testing and Materials (ASTM) D 7359 method, the chlorine (Cl) content was measured through elemental analysis of the isocyanate compositions prepared in Examples and Comparative Examples using a combustion ion chromatograph, and is shown in Table 2 below.

### <Measurement for APHA of XDI composition>

In accordance with the American Society for Testing and Materials (ASTM) E313 method, the APHA color of the isocyanate compositions prepared in Examples and Comparative Examples was measured using a spectrophotometer (Ultrascan Pro by HunterLab) (light source: C/2), and is shown in Table 2 below.

### <Abbe number of lens>

After bringing each flat lens manufactured using the isocyanate compositions of Examples and Comparative Examples into close contact with the measurement unit of ATAGO's Abbe refractometer (DR-M4), the refractive index of each was measured using C', F', and E' Filters, and the Abbe number (Ve) was measured according to Equation 1 below. [Equation 1] $Ve = \frac{ne - 1}{nF ′ - nC ′}$

In Equation 1,
Ve is the Abbe's number,
nₑ is the refractive index at the Fraunhofer e-line (wavelength 546.1 nm),
nF' is the refractive index at the Fraunhofer F'-line (wavelength 480.0 nm),
nC' is the refractive index at the Fraunhofer C'-line (wavelength 643.9 nm).

Here, it can be said that the higher the Abbe number, the higher the clarity of the lens.

### <Weather resistance evaluation of lens>

Each lens manufactured using the isocyanate compositions of Examples and Comparative Examples was exposed to acceleration conditions of a Xenon arc light source (irradiance 0.51W/m²@340nm, humidity 50%, temperature 60 °C) for 110 hours, and then Y.I values before and after exposure were compared.

After measuring the yellowness (YI, Yellowness) using a spectrophotometer (Ultrascan Pro by HunterLab) in accordance with the method of the American Society for Testing and Materials (ASTM) D1209, the weather resistance was evaluated by calculating the lens yellowness difference ΔYI before and after light source exposure according to Equation 2 below. [Equation 2] 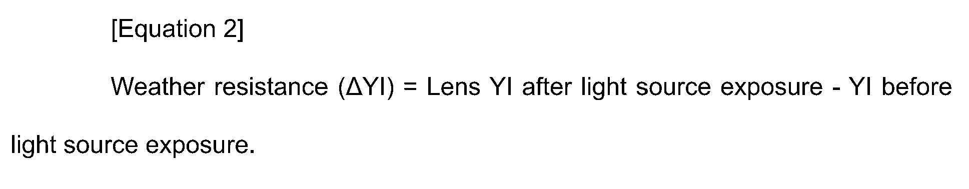 Weather resistance (ΔYI) = Lens YI after light source exposure - YI before light source exposure.

It can be said that the smaller the yellowness difference thus obtained, that is, the weather resistance ΔYI, the less the yellowing of the isocyanate (XDI) composition.

### <Striae evaluation of lens>

Each lens manufactured using the isocyanate compositions of Examples and Comparative Examples was observed with the naked eye through a Xenon lamp light source device, and the striae of the lens were evaluated in the following 3 stages.
- No striae: ⊚
- Slightly striae observed: ○
- Many striae: Δ.

### [Test Example 3]

For the isocyanate compositions of Examples and Comparative Examples, the XDI adducts manufactured using them, and the optical lenses, property evaluation was performed after storing for 12 months as follows, and the measurement results are shown in Table 2 below.

Specifically, the isocyanate compositions of Examples and Comparative Examples were stored in general tin cans under conditions of a temperature of 15 °C for 12 months. Then, using the isocyanate (XDI) composition after 12 months of storage, APHA measurement, YI measurement after lens preparation, and GC analysis of XDI after 12 months of storage were performed in the same manner as in Test Example 2.

In particular, when irradiating the XDI after 12 months of storage with a fluorescent lamp and a zirconium lamp, respectively, the presence or absence of cloudiness was visually checked, and lens preparation was not performed when cloudiness was confirmed.

### <Content of uretdione in XDI composition after 12 months of storage>

The content of uretdione in the XDI composition after 12 months of storage was measured by performing infrared spectroscopy (IR) analysis based on the preparation of uretdione XDI as follows.

### (1) Preparation of uretdione XDI

100 g of xylene diisocyanate (XDI) was added under dry nitrogen at room temperature, and 1 g of triisodecyl phosphite and 2 g of 4-dimethylaminopyridine were stirred as catalysts. After 20 hours, the reaction mixture was stopped with dibutyl phosphate and operated by thin film distillation. Uretdione XDI having the following Chemical Formula 5-1 obtained after thin film distillation was obtained.

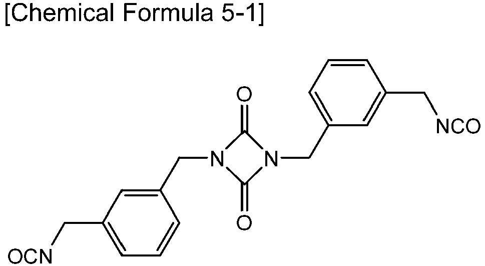

¹³C-NMR (100 MHz, CDCl₃) δ 156.6, 137.7, 135.9, 129.4, 127.4, 126.5, 126.1, 123.3, 46.2, 44.2.

### (2) Measurement of uretdione content in XDI composition after 12 months of storage

The XDI after 12 months of storage for Examples and Comparative Examples was measured by the ATR mode of an FT-IR spectrometer (Varian 4100).

The prepared uretdione XDI has an absorption peak of the uretdione carbonyl group (1770-1790 cm⁻¹). Using the prepared uretdione XDI as a standard substance, a calibration curve was prepared. In all infrared absorption spectra of XDI stored for 12 months, an absorption peak having the peak top of 1770 cm⁻¹ to 1790 cm⁻¹ appeared. The uretdione weight is calculated based on the absorption coefficient obtained from the calibration curve. And the content (%) of uretdione in the XDI composition is derived based on Equation 3 below. [Equation 3] $Uretdione \left(%\right) = Uretdione weight / Total weight of XDI composition \times 100 .$

### <IMBAl, IMBAm content ratio in 12-month XDI composition>

Isocyanomethylbenzaldehyde (IMBAl) of the following Chemical Formula 3-1 and isocyanomethylbenzamide (IMBAm) of the following Chemical Formula 4-1 generated during storage in the isocyanate compositions prepared in Examples and Comparative Examples, and their contents were derived by analysis through gas chromatography (GC).

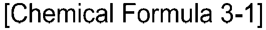

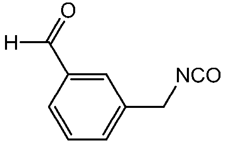

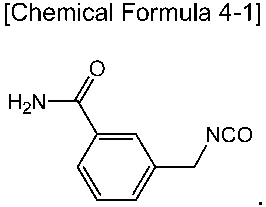

Specifically, as a result of performing under the following analysis conditions, the content ratio of each compound relative to the total weight of the isocyanate composition was represented, where the retention time of XDI represents a compound within 27.9 minutes to 29.0 minutes, IMBAI represents a compound with a retention time of 24.7 minutes to 26.4 minutes, and IMBAm represents a compound with a retention time of 30.7 minutes to 32.8 minutes. Here, the initial values of the content of each component measured in the isocyanate composition before long-term storage were IMBAI 0.007 wt%, IMBAm 0 wt%.

### 1. GC Measurement Conditions

Equipment: GC (Agilent HP-7890A FID)
Column: DB-17 (30 m x 0.25 mm, 0.5 µm)
Split ratio: 100:1
Inlet temperature: 250 °C
Detector temperature: 280 °C
Oven temperature: 80 °C → 5 °C/min ramp up, 160 °C (8 min hold) → 20 °C/min ramp up, 280 °C (18 min hold)
Carrier gas: N₂
Carrier gas flow rate: 1.0 mL/min (constant flow control)
Sample concentration: 1.0 wt% dichloromethane solution
Injection volume: 1.0 µL

For the isocyanate compositions of Examples and Comparative Examples, the XDI adducts manufactured using them, and the optical lenses, property evaluation was performed in the initial stage within 5 days after each preparation and after long-term storage of 12 months, and the measurement results are shown in Table 2.

**[Table 2]**

| | | Exampl e 1 | Exampl e 2 | Exampl e 3 | Compa rative Exampl e 1 | Compa rative Exampl e 2 | Compa rative Exampl e 3 |
|---|---|---|---|---|---|---|---|
| Initial properti es after manufac turing XDI composi tion, XDI adduct, and lens | DCMBI (ppm) | 68 | 100 | 150 | 5 | 40 | 330 |
| | Cl content (ppm) | 61 | 95 | 132 | 40 | 49 | 201 |
| | Lens Abbe number (Ve) | 31.4 | 31.7 | 31.8 | 31.3 | 31.2 | 32.2 |
| | Striae | ⊚ | ⊚ | ⊚ | ⊚ | ⊚ | ○ |
| | Lens weather resistance Δ YI | 0.47 | 0.47 | 0.53 | 0.55 | 0.55 | - |
| | Color after XDI adduct manufacturin g | Transp arent | Transp arent | Transp arent | Cloudin ess | Transp arent | Yellowi sh transpa rent |
| Properties after 12 months of storage | APHA of XDI composition | 18 | 16 | 14 | Visually cloudin ess | 30 | 37 |
| | Lens YI | 4.28 | 4.27 | 4.18 | - | 5.1 | 5.8 |
| | IMBAI content in XDI composition (wt%) | 0.13 | 0.09 | 0.06 | 0.3 | 0.25 | 0.05 |
| | IMBAm content in XDI composition (wt%) | 0.09 | 0.07 | 0.05 | 0.36 | 0.42 | 0.05 |
| | Uretdione content in XDI composition (wt%) | 2.7 | 1.2 | 0.1 | 10.1 | 5.3 | 0.02 |

As shown in Table 2 above, the isocyanate compositions of Examples 1, 2, and 3, in which DCMBI, which is a compound represented by Chemical Formula 1, is included in the isocyanate composition according to the present disclosure and its content is optimized to 68 ppm, 100 ppm, and 150 ppm, respectively, have improved Abbe numbers during lens manufacturing compared to Comparative Examples 1 to 3. The higher the Abbe number, the lower the dispersion, providing a clear image. Along with this, since the storage stability is improved even for a long time of several months or more, it has a significantly lower APHA value, so discoloration and cloudiness occurrence is suppressed, and the increase ratio of the content of impurities that can increase during storage in XDI and uretdione caused by self-polymerization of XDI is also suppressed, minimizing product deterioration for 12 months, and it can be confirmed that the yellowness of the optical lens can be greatly lowered.

On the other hand, when the content of DCMBI is significantly low at 5 ppm as in Comparative Example 1, stability decreases due to the heat exposure process during XDI adduct manufacturing, resulting in cloudiness, and haze occurred in the lens after 12 months. Even when the content of DCMBI is 40 ppm as in Comparative Example 2, the content is insufficient to maintain storage stability, so it could not maintain the color of XDI itself after storing for 12 months or more, and had an APHA of 30, and the lens also had a high yellowness with a YI of 5.1, and in addition, the contents of impurities such as IMBAl, IMBAm, and uretdione also showed relatively high figures, and in particular, it was confirmed that the content of uretdione increased significantly to 5.3 wt%. Also, when the content of DCMBI is excessively increased to 330 ppm as in Comparative Example 3, the Cl content of XDI itself is also as high as 201 ppm, and it also had a phenomenon of showing a yellowish color in the XDI adduct, and it can be confirmed that the YI is as high as 5.8 in the lens even when stored for 12 months or more.

Therefore, it can be seen that by optimizing the content of the compound (DCMBI) represented by Chemical Formula 1 in the isocyanate composition according to the present disclosure, the reactivity of xylene diisocyanate (XDI) itself can be controlled, thereby retaining excellent stability of XDI itself. In addition, due to this, the storage stability of the isocyanate composition is improved, so discoloration and cloudiness occurrence is suppressed, and it has an excellent effect of maintaining excellent discoloration resistance and improving transparency when applied to optical products.

## Claims

1. An isocyanate composition comprising xylylene diisocyanate and a compound represented by the following Chemical Formula 1, wherein the content of the compound represented by Chemical Formula 1 is greater than 60 ppm to 200 ppm or less: 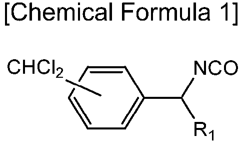 In Chemical Formula 1,
R₁ is hydrogen, methyl, or ethyl.

2. The isocyanate composition of Claim 1, wherein the content of the compound represented by Chemical Formula 1 is 62 ppm or more to 180 ppm or less.

3. The isocyanate composition of Claim 1, wherein the isocyanate composition has a chlorine (CI) content of 200 ppm or less based on the total weight of the isocyanate composition, measured using a combustion ion chromatograph in accordance with the American Society for Testing and Materials (ASTM) D 7359 method.

4. The isocyanate composition of Claim 1, wherein the isocyanate composition has an APHA color index of 0.1 or more to 25 or less, measured using a spectrophotometer (Ultrascan Pro by HunterLab, light source: C/2) in accordance with the American Society for Testing and Materials (ASTM) E313 method.

5. The isocyanate composition of Claim 1, further comprising at least one selected from the group consisting of chloromethylbenzyl isocyanate, isocyanomethylbenzaldehyde, and isocyanomethylbenzonitrile.

6. The isocyanate composition of Claim 1, wherein the content of uretdione measured after 12 months of storage is 5 parts by weight or less based on 100 parts by weight of the isocyanate composition.

7. A polymerizable composition comprising:
the isocyanate composition of Claim 1; and
at least one selected from the group consisting of a polyfunctional thiol-based compound, a polyfunctional alcohol-based compound, and a polyfunctional episulfide-based compound.

8. The polymerizable composition of Claim 7, further comprising at least one selected from the group consisting of a polymerization initiator, an internal release agent, and an ultraviolet absorber.

9. An optical article comprising a polymer obtained by polymerizing the polymerizable composition of Claim 7.

10. The optical article of Claim 9, wherein the article is an optical pressure-sensitive adhesive, an optical adhesive, an optical coating, or an optical lens.
